(19) Europäisches Patentamt
European Patent Office
Office européen des brevets

(11) **EP 3 599 615 A1**

(12) **EUROPEAN PATENT APPLICATION**

(43) Date of publication:
**29.01.2020   Bulletin 2020/05**

(21) Application number: **18306024.3**

(22) Date of filing: **27.07.2018**

(51) Int Cl.:
***G16H 50/20*** (2018.01)     ***G16H 50/30*** (2018.01)
***G01N 33/68*** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Biopredictive
75007 Paris (FR)**

• **Assistance Publique - Hôpitaux de Paris
75004 Paris (FR)**

(72) Inventor: **POYNARD, Thierry
75006 Paris (FR)**

(74) Representative: **Flesselles, Bruno F.G.
BF IP
36 rue Jean de la Fontaine
75016 Paris (FR)**

(54) **METHOD OF DIAGNOSIS OF LIVER STEATOSIS**

(57)    The present invention relates to new methods for assessing the presence of nonalcoholic steatohepatitis (steatosis) in a patient, using functions combining biological markers without bilirubin and Body Mass Index being used as markers in the function.

**Construction and Validation of SteatoTest-2**

Figure 1

**Description**

The invention relates to a new non-invasive quantitative test making it possible to detect liver steatosis and help identify subjects at risk of non-alcoholic fatty liver disease (NAFLD) and in particular those with non-alcoholic steatohepatitis (NASH) or fibrosis.

Fatty liver is a reversible condition wherein large vacuoles of triglyceride fat accumulate in liver cells via the process of steatosis.

Non-alcoholic fatty liver disease (NAFLD) is one of the causes of fatty liver, occurring when fat is deposited (steatosis) in the liver due to causes other than excessive alcohol use. NAFLD is the most common liver disorder in developed countries. NAFLD is a condition generally associated with factors of the metabolic syndrome.

While a liver may remain fatty without disturbing liver function, it may also progress to become non-alcoholic steatohepatitis (NASH), a state in which steatosis is combined with inflammation and fibrosis (steatohepatitis). NASH is a progressive disease: over a 10-year period, up to 20% of patients with NASH will develop cirrhosis of the liver, and 10% will suffer death related to liver disease.

Non-alcoholic steatohepatitis (NASH) is thus the most extreme form of NAFLD, and is regarded as a major cause of cirrhosis of the liver of unknown cause.

Biopsy is the usual reference for liver steatosis assessment. Whenever imaging tools (ultrasonography, MRE), are not available or feasible (e.g. large epidemiological studies), serum biomarkers and scores are an acceptable alternative for the diagnosis of steatosis.

The European guidelines have recommended three blood tests for the diagnosis of steatosis in large studies of subjects at risk of non-alcoholic fatty liver disease (NALFLD) and NASH (EASL J Hepatol 2015). The best-validated steatosis scores are the fatty liver index, the SteatoTest (Poynard et al 2005, Comparative Hepatology 4:10) and the NAFLD liver fat score (Angulo et al, Hepatology 2007;45(4):846-854); they have all been externally validated in the general population or in grade 3 obese persons and variably predict metabolic, hepatic and cardiovascular outcomes/mortality. These scores are associated with insulin resistance and reliably predict the presence, not the severity, of steatosis (Fedchuk et al, Aliment Pharmacol Ther 2014;40:1209-1222).

The SteatoTest is calculated using an original combination of nine biochemical markers, which are easy to assess and combines alpha2-macroglobulin, haptoglobin, apolipoprotein A1, total bilirubin, Gamma-glutamyl transpeptidase (GGT), fasting glucose, triglycerides, cholesterol, and alanine transaminase (ALT), with parameters adjusted for patient's age, gender, weight and height (Body Mass Index).

Among the SteatoTest 9 serum components, two are two sources of variability that may hamper the reliability of the result: the body mass index (BMI) and the total bilirubin.

The NAFLD liver fat score uses age, BMI, IGF/ Diabetes (Yes/No), platelets, AST, ALT, and albumin (NAFLD Score = -1.675 + 0.037 × Age (years) + 0.094 × BMI (kg/m$^2$) + 1.13 × IFG/diabetes (yes = 1, no = 0) + 0.99 × AST/ALT ratio- 0.013 × Platelet (×10$^9$/L) - 0.66 × Albumin (g/dL)).

These two tests use BMI as a variable in the formulas. However, Butler et al (2017, Clinical Nutrition ESPEN 22, 112e115) and Sorkin et al (1999, American Journal of Epidemiology, 150(9), 969-977) show that there is some variability in the BMI calculations. In particular, there is a decrease in height of people over the years that is usually not taken into consideration when such information is asked to the patients. Furthermore, people may not know (or give) their exact weight. Altogether, this implies that the calculated Body Mass Index (BMI (kg/m$^2$) = weight (kg) / height (m)$^2$) is usually not accurate.

Furthermore, Poynard et al (BMC Gastroenterol. 2011; 11: 39) show that using bilirubin could induce false positive due in particular in patient with Gilbert syndrome or hemolysis.

There is thus a need to develop new tests to detected liver steatosis, in particular when associated with inflammation (NASH) or fibrosis, in to simplify the feasibility of such multi-analytes biomarker. This test should preferably be of the same quality than the tests of the prior art (Steatotest).

The quality of a test is generally determined by drawing a Receiving Operating Characteristic (ROC) curve and measuring the Area Under Receiving Operating Characteristic curve (AUROC).

The ROC curve is drawn by plotting the sensitivity versus (1-specificity), after classification of the patients, according to the result obtained for the test, for different thresholds (from 0 to 1).

It is usually acknowledged that a ROC curve, the area under which has a value superior to 0.7, is a good predictive curve. The ROC curve has to be acknowledged as a curve allowing prediction of the quality of a test. It is best for the AUROC to be as closed as 1 as possible, this value describing a test which is 100 % specific and sensitive.

It is reminded that

(1) sensitivity is the probability that the diagnosis is positive in individuals having the phenotype sought (detection of true positives): the test is positive if the patient is having the phenotype. The sensitivity is low when the number of false negatives is high. The sensitivity is calculated by the formula SE = (number of individuals having the phenotype in whom the sign is present)/(number of individuals having the phenotype in whom the sign is present + number of individuals having the phenotype in whom the sign is absent).

(2) specificity is the probability that the diagnosis is negative in the individuals not having the phenotype sought (non-detection of true negatives): the test is negative if the patient is not suffering from the disease. The specificity is low when the number of false positives is high. The specificity is calculated by the formula SP = (number of individuals not having the phenotype in whom the sign is absent)/(number of individuals not having the phenotype in whom the sign is absent + number of individuals not having the phenotype in whom the sign is present).

(3) Positive predictive value (PPV): is the probability of having the disease if the diagnostic test is positive (i.e. that the patient is not a false positive): the patient is having the phenotype if the test is positive. The positive predictive value is calculated by the formula PPV = (number of individuals having the phenotype in whom the sign is present)/(number of individuals having the phenotype in whom the sign is present + number of individuals not having the phenotype in whom the sign is present).

(4) Negative predictive value (NPV): is the probability of not having the disease if the diagnostic test is negative (that the patient is not a false negative): the patient is not having the phenotype if the test is negative. The negative predictive value is calculated by the formula NPV = (number of individuals not having the phenotype in whom the sign is absent)/(number of individuals not having the phenotype in whom the sign is absent + number of individuals having the phenotype in whom the sign is absent)

In order to obtain a good diagnostic test, it is important to both increase specificity and sensitivity.

Generally, a diagnosis method comprises

i. a step of gathering information from the patient
ii. a step of comparing said information with regards to thresholds
iii. a step of deducing, from the difference between the patient's information and the threshold, whether the patient has a specific disease, the stage of the patient's disease, or whether the patient's state will evolve to a given state.

As a matter of illustration

i. the information that can be gathered from the patient can be gathered directly from the patient (such as images from NMR, scanner, radiography, contrast-enhanced computed tomography), or indirectly from the patient, such as from a biological sample that has been obtained from a patient (such as urine, blood sample...). The information can be presence (or absence) and/or level of specific biological markers, whether specific from the pathogenic determinant (bacterial or viral DNA/RNA), or elevated levels of patient's markers
ii. once the information is obtained, it is compared to different values / standards and the deviation with regards to these standards is assessed. As a matter of illustration, the level of some biomarkers shall be compared to the level usually observed in healthy patients and to the levels usually observed in patients with the disease (or for patients who have been known to later evolve to a specific disease stage, for prognosis methods). Thresholds may exist, where 95 % of patients having passed the threshold have the disease and 95 % of the patients not having passed the threshold do not have the disease. For diseases where multiple clinical stages can be determined, such thresholds can discriminate the different stages. In this step ii, one may compare various types of information to their respective standards, in order to be able to reach a diagnostic in step iii (as a matter of illustration, one can use the values and information obtained from measurement of various blood or plasma markers, images from scanner and of Body Mass Index).
iii. the last step is actually making the diagnosis (resp. determining a prognosis) *i.e.* deciding whether or not the patient has the condition sought resp. whether or not the patient will evolve to a given clinical state), taking, in particular, into account the information gathered from the patient, the thresholds as described above. The physician may also take into account other elements (such as the consistency of the information gathered or the like) to make the diagnostic.

Some methods, such as the ones disclosed in the present application, shall also include a step i.a), which comprise the steps of modifying the information obtained from the patient in order to obtain a new type of information, which is the one that is then compared to the standards in step ii. Such modification is the combination of the values of variables in a function, and obtaining an end value.

It is further to be noted that the mere measurement of the values of levels of markers in the plasma or serum of a patient and the combination thereof in an algorithm as herein disclosed is part of a method but only provides an intermediate result (an end value or index) that would then to be compared to a reference index (threshold), in order to actually be able to pose the diagnostic.

It is also to be noted that the tests herein disclosed are not "gold-standard" tests, in the sense that the output (index calculated by the formulas herein disclosed) isn't a definitive answer as to the state of the patient. Indeed, these tests are based on statistics and there may thus be false-positive or false-negative results, which is the reason why the specific experience of the physician in interpreting the index is of importance for making the prognosis and deciding which kind of follow up is to be made to ne made for each patient.

However, due to the specificity, sensitivity, positive predictive value and negative predictive value of the tests, herein provided for various thresholds of the index, these tests are of great interest in provided a help to the physician when investigating a clinical case. Consequently, step iii as disclosed above is not direct and immediate from step ii, as the physician must interpret the result from the clinical and general context to be able to reach a conclusion.

The invention thus relates to an *in vitro* method for diagnosing the presence of steatosis in a subject comprising the step of

(a) combining the values of at least three biological markers, selected from the group consisting of:

◦ the amount of biochemical markers as measured circulating in the blood, serum or plasma, of the subject, and
◦ physical characteristics of a patient

in a mathematical function, in order to obtain an end value.

The function may also include the stepf of:

(b) optionally comparing the end value to predetermined values, and
(c) determining presence of steatosis based on the end value calculated on (a),

with the proviso that Body Mass Index and level of bilirubin in the subject are not used as biological markers in (a).

This method is performed *in vitro, or ex vivo.* It is preferred when the values of at least three, preferably at least four, preferably at least five, preferably at least or exactly six, preferably at least or exactly seven, preferably at least or exactly eight, preferably at least or exactly nine biological markers are combined in the function.

Below are described a few functions that can be used for performing the method herein disclosed. It is however to be noted that there is no technical difficulty to obtain and develop other functions that would be as (or more) efficient as the functions herein disclosed, following the teachings of the invention, and avoiding use of bilirubin and BMI in the markers and variables used.

It is also to be noted that, although the function herein exemplified have been obtained by logistic regression, other statistical methods could be used to provide a function that would use the values of various markers and provide a value indicative of the presence of steatosis.

Although any markers can be used in the disclosed function, one shall avoid using bilirubin (total bilirubin) and BMI in the function, for the reasons disclosed above.

The predetermined values are calculated and determined by the person skilled in the art, depending on the actual function. It is preferred to have a first predetermined value under which the Negative Predictive Value is higher than 90%, preferably higher than 95 %, more preferably higher than 98 %. Consequently, any end value lower than this first predetermined value will mean that the patient has no steatosis. It is preferred to normalize the function so that this first predetermined value is 0.25.

It is also possible to define a second predetermined value above which the Positive Predictive Value is higher than 75 %, preferably higher than 85 %, more preferably higher than 90 %. Consequently, any end value higher than this second predetermined value will mean that the patient has steatosis and that NASH and fibrosis, two severe complications of steatosis should be diagnosed. It is preferred to normalize the function so that this second predetermined value is 0.50.

The present method uses a semi-quantitative function that makes it possible to provide information about steatosis in the liver of the patient. This means that the higher the end value, the more steatosis in the liver of the patient.

The method shall be used by the physician as follows:

When the test is positive (i.e. when the end value is higher than a predetermined threshold), the physician will first search for any other causes of liver disease that could induce steatosis and that can be easily detected, namely whether the patient has alcoholic steatosis the most frequent, and viral hepatitis with specific marker (hepatitis B,C), drug induced hepatitis or genetic steatosis or infectious steatosis. If this potential cause of steatosis is discarded, the physician will look at other metabolic causes (such as overweight, type 2-diabetes, dyslipidemia, high blood pressure, hyperuricemia...). The existence of such other cause makes it possible to finalize the diagnosis, as these causes are associated with increased fat in the liver.

It is also to be noted that the patient will generally consult the hepatologist after some abnormalities have been detected (such as abnormalities in the common liver markers, or detection of fat in the liver by echography), so that the diagnosis will be easier to be made.

The method herein disclosed will thus generally be performed with other existing tests (the Fibrotest, disclosed in WO0216949, and NASH test disclosed in WO2018050804. Having the results of all these tests will allow the physician to make an accurate diagnosis about the liver pathology of the patient.

In summary, the end value allows to determine presence of steatosis, also taking into account the general condition and clinical assessment of the patient, which is always to take into account for diagnosis methods. The method thus makes it possible to conclude about the presence or absence of steatosis in the patient, and/or to diagnose the presence of associated inflammation (NASH) or fibrosis.

This method is particularly interesting to detect absence of steatosis (when the end value is below the first predetermined value) as this allows to avoid any unnecessary exams for the patient and will guide the physician to investigate other causes if needed.

In the method, one would prefer one or more of the following, in either combination:

(a) Bilirubin is not included in the biochemical markers the concentration of which is measured in step (a).

(b) The biochemical markers of step (a) are selected from the group consisting of α2-macroglobulin (A2M), GGT (gammaglutamyl transpeptidase), haptoglobin, apolipoprotein A-I (apoA1), alanine transaminases (ALT), aspartate transaminases (AST), triglycerides, total cholesterol, fasting glucose, γ-globulin, albumin, α1-globulin, α2-globulin, β-globulin, IL10, TGF-β1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1 and carbohydrate deficient transferrin

(c) The biochemical markers of step (a) are selected from the group consisting of α2-macroglobulin, AST (aspartate aminotransferase), ALT (alanine aminotransferase), GGT (gammaglutamyl transpeptidase), total bilirubin, haptoglobin, apoA1, triglycerides, total cholesterol, fasting glucose, γ-globulin, albumin, α1-globulin, α2-globulin, β-globulin, IL10, TGF-β1, apoA2, apoB, cytokeratin 18 and cytokeratin 19 components, platelets number, prothrombin level, hyaluronic acid, urea, N-terminal of type III pro-collagen, Tissue inhibitor metalloproteinase type-1 (TIMP-1), type IV collagen (Coll IV) and osteoprotegerin

(d) The biochemical markers of step (a) are Alpha-2-macroglobulin, ApoA1, GGT, Haptoglobin, ALT, AST, fasting glucose, total cholesterol, and triglycerides.

(e) at least 6, more preferably at least 7, more preferably at least 8, more preferably at least or exactly 9, biochemical markers are used in the mathematical function

(f) The function of a) further includes at least one, and preferably both, physical characteristic of the patient selected from the group consisting of gender and age of the patient

(g) BMI is not used as a physical characteristic of the patient

(h) The function of (a) includes the values measured for circulating Alpha-2-macroglobulin, ApoA1, GGT, Haptoglobin, ALT, AST, fasting glucose, total cholesterol, triglycerides, as well as the values corresponding to the age and sex of the patient.

(i) The function of a) combines the measured values of Alpha-2-macroglobulin, ApoA1, GGT, Haptoglobin, ALT, AST, fasting glucose, total cholesterol, and triglycerides, as well as the age and gender of the patient.

(j) The function is a logistic function, i.e. a function that has been obtained by logistic regression, as explained below

(k) The function is a semi-quantitative function.

In a particular embodiment, the function has been obtained by:

a) evaluating the presence of steatosis in a cohort of patients, wherein the values of circulating biochemical markers are known for the patients

b) identifying by unidimensional analysis, the circulating biochemical markers for which the values differ significantly between the groups of

i. patients with steatosis and

ii. patients without steatosis

c) performing a logistic regression analysis to assess and ponder the independent discriminative value of the markers identified in step b) for the occurrence of steatosis

d) thereby obtaining the function, by combination of these identified independent factors.

A further explanation of this process used to obtain the diagnosis function is developed below.

In the preferred embodiment, the function is a0 + a1 x Age (years) + a2 x ApoA1 (g/l) + a3 x Log (A2M, g/l) + a4 x Log(GGT, IU /l) + a5 x Log(ALT, IU /l) + a6 x Log(AST, IU /l) + a7 x Log (Hapto, g/l) + a8 x Log (Triglycerides TG, mmol/l) + a9 x Log (Total Cholesterol CT, mmol/l) + a10 x Log (Fasting glucose, g/l) + a11 x Gender (0 for women, 1 for men).

In particular,

a) $4.8 \leq a0 \leq 5.5$ preferably $5 \leq a0 \leq 5.3$

b) $-0.03 \leq a1 \leq -0.015$ preferably $-0.025 \leq a1 \leq -0.02$

c) $0.9 \leq a2 \leq 1.2$ preferably $0.95 \leq a2 \leq 1.05$

d) $1.8 \leq a3 \leq 2.2$ preferably $1.95 \leq a3 \leq 2.05$

e) $-1.3 \leq a4 \leq -1.1$ preferably $-1.2 \leq a4 \leq -1.1$

f) $-1.45 \leq a5 \leq -1.2$ preferably $-1.36 \leq a5 \leq -1.3$

g) $0.5 \leq a6 \leq 0.7$ preferably $0.55 \leq a6 \leq 0.65$

h) $-0.35 \leq a7 \leq -0.22$ preferably $-0.3 \leq a7 \leq -0.25$

i) $-1.45 \leq a8 \leq -1.25$ preferably $-1.4 \leq a8 \leq -1.3$

j) $-0.8 \leq a9 \leq -0.6$ preferably $-0.75 \leq a9 \leq -0.65$

k) $-3.55 \leq a10 \leq -3.35$ preferably $-3.5 \leq a10 \leq -3.4$

l) $0.35 \leq a11 \leq 0.55$ preferably $0.4 \leq a11 \leq 0.5$.

In the preferred embodiment, the function is 5.17 - 0.022 x Age (years) + 1.02 x ApoA1 (g/l) + 1.99 x Log (A2M, g/l) - 1.16 x Log(GGT, IU / I) - 1.33 x Log(ALT, IU / I) + 0.6 x Log(AST, IU / I) - 0.29 x Log (Hapto, g/l) - 1.35 x Log (Triglycerides TG, mmol/l) - 0.68 x Log (Total Cholesterol CT, mmol/l) - 3.46 x Log (Fasting glucose, g/l) + 0.46 x Gender (0 for women, 1 for men).

The coefficients of the formulas as provided above have been rounded to the 2nd decimal, and any function using coefficients rounded to a further or lower decimal can also be used as herein disclosed and would thus be equivalent.

As an illustration, a function with coefficients rounded to the 5th decimal could be 5.17005 - 0.02202 x Age (years) + 1.01850 x ApoA1 (g/l) + 1.98726 x Log (A2M, g/l) - 1.16378 x Log(GGT, IU / I) - 1.32517 x Log(ALT, IU / I) + 0.60003 x Log(AST, IU / I) - 0.28781 x Log (Hapto, g/l) - 1.34979 x Log (Triglycerides TG, mmol/l) - 0.68426 x Log (Total Cholesterol CT, mmol/l) - 3.45945 x Log (Fasting glucose, g/l) + 0.46473 x Gender (0 for women, 1 for men). Such function is also subject of the invention.

The method herein disclosed thus makes use of an *ex vivo* combination of value of different variables, in order to obtain an end-result that is indicative of the presence of steatosis in a patient. It would thus exclude any step applied on the human body of the patient. In the method herein disclosed, it is understood that the markers, the value of which is measured and used as variable in the function, are circulating proteins or natural elements that are present in the blood, plasma or serum of a patient. The values of the chosen markers shall be measured on a sample of blood, plasma or serum previously harvested, according to methods known in the art. The values are expressed in the units according to the art. However, should other units be chosen by the person skilled in the art to expressed the measured values, this would only change the coefficients within the logistic function. The method would thus still be applicable.

However, in another embodiment, the invention relates to a method for prognosis of the presence of steatosis in a patient, comprising the step of harvesting blood, plasma or serum from a patient, measuring the value of markers present in the harvested sample and combining the values as measured through a function as disclosed above. The markers are as disclosed above. Thus, said method may also include the steps of obtaining (measuring) the different values used in the logistic function, such as the steps of measuring, in a blood or a plasma sample that have previously been harvested from the patient, the concentration / values / quantities of the various biological markers as mentioned above.

Said method may also include the steps of comparing the first index to a predetermined threshold, in particular to determine whether it is higher or lower than said threshold.

Said method may also include the step of deducing the presence of steatosis in said patient if the first index is above said threshold.

The test as designed here is a quantitative test, the end result value ranging from 0 to 1. It is considered that a patient is likely to present steatosis (as explained above) and would thus need a treatment or follow-up when the end result is equal or above 0.25.

In the above-specified methods, the first predetermined value has been designed to be 0.25, and the patient has nonalcoholic steatohepatitis (steatosis) if the end result is higher or equal to 0.25.

In brief, for an end value under 0.25, the patient has no steatosis. For an end value between 0.25 and 0.50, there needs to be a follow-up for the patient with new evaluations on a regular basis. For an end value between 0.50 and 0.75, there is clinical significance and a treatment should be made (starting with diet and exercise). Above 0.75, the steatosis is quite developed and strong treatment should be started, the patient having likely other hepatic problems.

The invention also includes a device for diagnosis of steatosis in a patient, comprising:

a) a first means, wherein the first means provides a first index by combining the values as measured from markers present in the serum or plasma of a patient through a logistic function, wherein said first logistic function is

$$a1 + a2 \times \mathrm{Log}\,(A2M, g/l) + a3 \times \mathrm{Age\,(years)} + a4 \times \mathrm{Log\,(ALT, IU\,/\,l)} + a5 \times (Apoa1, g/l) + a6 \times \mathrm{Log\,(AST, IU/l)} + a7 \times \mathrm{Log\,(BILI, \mu mol/l)} + a8 \times \mathrm{Log\,(CT, mmol/l)} + a9 \times \mathrm{Gender\,(0\ for\ women,\ 1\ for\ men)} + a10 \times \mathrm{Log(GGT, IU\,/\,l)} + a11 \times \mathrm{Log\,(Hapto, g/l)} + a12 \times \mathrm{Log\,(TG, mmol/l)}$$

With

- $-8 \leq a1 \leq -7$
- $0.1 \leq a2 \leq 0.6$ preferably $0.15 \leq a2 \leq 0.55$
- $0.02 \leq a3 \leq 0.05$ preferably $0.03 \leq a3 \leq 0.04$
- $1.1 \leq a4 \leq 1.5$ preferably $1.2 \leq a4 \leq 1.4$
- $-0.2 \leq a5 \leq 1.0$
- $1.8 \leq a6 \leq 2.3$ preferably $1.95 \leq a6 \leq 2.2$
- $0.8 \leq a7 \leq 1.6$ preferably $0.9 \leq a7 \leq 1.5$
- $-1.7 \leq a8 \leq -1.3$ preferably $-1.6 \leq a8 \leq -1.4$
- $0.015 \leq a9 \leq 0.20$
- $0.15 \leq a10 \leq 0.25$ preferably $0.20 \leq a10 \leq 0.22$
- $-03 \leq a11 \leq 0.1$
- $0.9 \leq a12 \leq 1.2$ preferably $1.0 \leq a12 \leq 1.1$.

Said first index can be later used to determine the presence of steatosis in said patient and whether it is necessary to initiate treatment or follow-up, in particular using the help of table 1 above.

In a specific embodiment, the first mean is computerized. It may be an electronic spreadsheet with the formula recorded within, that provides the first index as an output when entering the various elements mentioned above. It can also be a computer program that provides the first index as an output after receipt of the various elements mentioned above.

The first means can present one or more of the following, in either combination:

- Operate within a private or public network
- Receive the inputs (values of the various elements mentioned above) from a sender that is in a remote place (i.e. they are sent to the first means from a different location that where the first means is located)
- Require the sender to identify himself before sending the inputs
- Receive the inputs (values of the various elements mentioned above) from a secure manner
- Send the output (first index) to the sender of the inputs
- Store the output in a database (possibly with a unique identifier, making it possible to assign inputs, outputs to this identifier)
- Provides the first index with further information (such as sensitivity and/or specificity and/or positive predictive value and/or negative predictive value linked to the prevalence of the condition in the population to which belongs the patient)

Is also foreseen a non-transitory computer readable storage medium, having stored thereon a computer program comprising program instructions, the computer program being loadable into a data-processing unit and adapted to cause the data-processing unit to carry out a method for calculating a first index by combining the values as measured from markers present in the serum or plasma of a patient through a logistic function as disclosed above, when the computer program is run by the data-processing device.

In another embodiment, the invention relates to a microprocessor comprising a computer algorithm to perform a method of determining the presence of steatosis in a patient: providing the values of blood markers of a patient and optionally the age and a value allocated to the sex of the patient; and performing a mathematical function (as disclosed above, or obtained as disclosed above) to combine the values to obtain a score useful for presence of steatosis in the patient.

The invention also relates to the use of the functions, devices and/or microprocessors in order to make a diagnosis of presence of steatosis in a patient by use of the functions and comparing the result to a predetermined threshold to determine the presence of steatosis in the patient.

The invention also relates to an ex vivo method for diagnosis a liver disease in a patient, comprising the steps of

- Performing the method herein disclosed to determine presence of steatosis in a patient
- Determining the presence of liver fibrosis in the patient.
- Determining the presence of liver inflammation in the patient, and

diagnosing a liver disease in the patient depending on the results of the above determinations.

It is preferred when liver fibrosis is determined by the combination of the values biological markers in a function, in particular by the Fibrotest. In other embodiments, liver fibrosis is determined by *ex vivo* measurement of liver stiffness by Vibration Controlled Transient Elastography.

It is preferred when presence of liver inflammation in the patient is determined by the combination of the values biological markers in a function, in particular by the NASH test.

Devices for performing the method are also subject of the invention.

In particular, the invention also relates to methods and device to detect a liver disease in a patient, comprising the steps of

- performing the method for detecting steatosis herein disclose,
- performing the method for determining liver fibrosis (Fibrotest) disclosed in WO0216949,
- performing the method for determining inflammation (NASH test disclosed in WO2018050804),

and concluding about the liver disease depending on the results obtained for each method.

The Fibrotest® combines five markers: alpha2-macroglobulin, haptoglobin, apolipoprotein A1, total bilirubin and gamma-glutamyl transpeptidase (GGT), and adjusts with sex and age. The algorithm for Fibrotest® reads as follows: 4.467 x Log(Alpha2Macroglobulin (g/l)) - 1.357 x Log(Haptoglobin (g/l)) + 1.017 x Log(GGT (IU/l)) + 0.0281 x Age (in years) + 1.737 x Log(Bilirubin ($\mu$mol/l)) - 1.184 x ApoA1 (g/l) + 0.301 x Sex (female=0, male=1) -5.540. It allows to determine the state of fibrosis (F2 or higher according to the METAVIR classification).

The NASH test is in the form of a1 + a2 x Log (A2M, g/l) + a3 x Age (years) + a4 x Log (ALT, IU /l) + a5 x (Apoa1, g/l) + a6 x Log (AST, IU/l) + a7 x Log (BILI, $\mu$mol/l) + a8 x Log (CT, mmol/l) + a9 x Gender (0 for women, 1 for men) + a10 x Log(GGT, IU /l) + a11 x Log (Hapto, g/l) + a12 x Log (TG, mmol/l) with

- -8 $\leq$ a1 $\leq$ -7
- 0.1 $\leq$ a2 $\leq$ 0.6 preferably 0.15 $\leq$ a2 $\leq$ 0.55
- 0.02 $\leq$ a3 $\leq$ 0.05 preferably 0.03 $\leq$ a3 $\leq$ 0.04
- 1.1 $\leq$ a4 $\leq$ 1.5 preferably 1.2 $\leq$ a4 $\leq$ 1.4
- -0.2 $\leq$ a5 $\leq$ 1.0
- 1.8 $\leq$ a6 $\leq$ 2.3 preferably 1.95 $\leq$ a6 $\leq$ 2.2
- 0.8 $\leq$ a7 $\leq$ 1.6 preferably 0.9 $\leq$ a7 $\leq$ 1.5
- -1.7 $\leq$ a8 $\leq$ -1.3 preferably -1.6 $\leq$ a8 $\leq$ -1.4
- 0.015 $\leq$ a9 $\leq$ 0.20
- 0.15 $\leq$ a10 $\leq$ 0.25 preferably 0.20 $\leq$ a10 $\leq$ 0.22
- -0.3 $\leq$ a11 $\leq$ 0.1
- 0.9 $\leq$ a12 $\leq$ 1.2 preferably 1.0 $\leq$ a12 $\leq$ 1.1

This NASH test allows to detect inflammation when the end result is higher than 0.25.

These tests are known in the art and their use is not further developed.

As indicated above, the function used in the method herein disclosed can be a function obtained by logistic regression (which can be called logistic function).

Methods to perform logistic regressions are known in the art. In summary, they consist in evaluating the individual variations of markers between populations of subjects (one with the output such as steatosis and the other where the output is absent). The markers which are the most variable can be chosen and logistic regression analysis is made to ponder the independent discriminative value of the selected markers. If some markers don't vary between groups, the coefficients of these markers in the function will be low (thereby indicating that the weight of these markers is of no real influence for the presence of the trait).

The function can then be normalized (for example to have the end result always comprised between 0 and 1).

The invention thus also pertains to a method for obtaining a function for identifying the presence (or absence) of steatosis (or diagnosing steatosis) in a patient wherein said function combines the values of the concentration of biochemical markers in the blood / serum or plasma of said patient and optionally the age, gender of the patient, comprising the steps of:

a) evaluating the presence of steatosis in a cohort of patients, wherein the values of circulating biochemical markers are known for the patients

b) identifying by unidimensional analysis, the circulating biochemical markers for which the values differ significantly between the groups of

i. patients with steatosis and
ii. patients without steatosis

c) identifying whether the age, gender of the patient differ significantly between the groups of

i. patients with steatosis and
ii. patients without steatosis

d) performing a logistic regression analysis to assess and ponder the independent discriminative value of the markers identified in step b) and c) for the occurrence of steatosis, wherein Body Mass Index and level of bilirubin are not incorporated in the list of markers from which the logistic regression is performed.

e) thereby obtaining the function, by combination of these identified independent factors, wherein the function allows for diagnosing the presence of nonalcoholic steatohepatitis (steatosis) in a subject and doesn't use the values of bilirubin or of BMI.

In the method for developing such diagnostic test, one would prefer one or more of the following, in either combination:

(a) in step a), to use a test cohort comprising at least 100 patients.
(b) In the cohort of a), that at least 50% of the patients should be presenting at least one factor of metabolic syndrome (and thus at risk of having steatosis), and not an exclusion factor (chronic or acute liver disease)
(c) In the cohort of a), that at least 10% of the patients are patients who have no steatosis and no activity
(d) The biochemical markers of step (a) are selected from the group consisting of α2-macroglobulin (A2M), GGT (gammaglutamyl transpeptidase), haptoglobin, apolipoprotein A-I (apoA1), alanine transaminases (ALT), aspartate transaminases (AST), triglycerides, total cholesterol, fasting glucose, γ-globulin, albumin, α1-globulin, α2-globulin, β-globulin, IL10, TGF-β1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1 and carbohydrate deficient transferrin
(e) The biochemical markers of step (a) are selected from the group consisting of α2-macroglobulin, AST (aspartate aminotransferase), ALT (alanine aminotransferase), GGT (gammaglutamyl transpeptidase), total bilirubin, haptoglobin, apoA1, triglycerides, total cholesterol, fasting glucose, $\gamma$-globulin, albumin, $\alpha$1-globulin, $\alpha$2-globulin, $\beta$-globulin, IL10, TGF-$\beta$1, apoA2, apoB, cytokeratin 18 and cytokeratin 19 components, platelets number, prothrombin level, hyaluronic acid, urea, N-terminal of type III pro-collagen, Tissue inhibitor metalloproteinase type-1 (TIMP-1), type IV collagen (Coll IV) and osteoprotegerin

(f) The biochemical markers of step (a) are Alpha-2-macroglobulin, ApoA1, GGT, Haptoglobin, ALT, AST, fasting glucose, total cholesterol, and triglycerides.

(g) at least 6, more preferably at least 7, more preferably at least 8, more preferably at least or exactly 9, biochemical markers are used in the mathematical function

(h) The function of obtained at the end includes at least one, and preferably both, physical characteristic of the patient selected from the group consisting of gender and age of the patient

(i) The function includes the values measured for circulating Alpha-2-macroglobulin, ApoA1, GGT, Haptoglobin, ALT, AST, fasting glucose, total cholesterol, triglycerides, as well as the values corresponding to the age and sex of the patient.

(j) The function combines the measured values of Alpha-2-macroglobulin, ApoA1, GGT, Haptoglobin, ALT, AST, fasting glucose, total cholesterol, and triglycerides, as well as the age and gender of the patient.

(k) The method further comprises a step of validating the logistic function on a validation cohort comprising at least 100 patients.

In order to obtain a function that is as accurate as possible, the number of patients in the cohort should be as large as possible, indicating that it preferably comprises more than 50 patients, preferably more than 100 patients, preferably more than 200 patients, more preferably more than 500 patients, or even more than 1000 patients. As indicated, there is no upper limit for the number of patients, and the larger, the better.

Once steatosis has been diagnosed in a patient, the physician can start and provide an appropriate diagnostic of associated severe disease, inflammation (NASH) or fibrosis. The invention also relates to a method for treating a patient having a NASH, for which there is a need to confirm presence of steatosis, comprising the steps of performing the method of diagnosis as disclosed above and providing the patient with appropriate treatment depending on its level of steatosis. In another embodiment, the invention provides a method for treating a patient, comprising the steps of providing appropriate steatosis treatment to a patient depending on its level of steatosis. Such treatment should in particular be performed when the end value calculated by the method as disclosed above is higher than 0.25, preferably higher than 0.50.

The primary treatment for patient with steatosis will be to a dietary treatment in order to manage obesity, high blood sugar, high blood lipids and excess iron levels if present. Alcohol should also be avoided. It is also intended to have a weight loss of the patient.

Medicinal drug can also be provided to the patient when steatosis is associated with NASH or fibrosis. As an illustration, one can cite pioglitazone, a medicine for type 2 diabetes, in particular in NASH patients who don't have diabetes.

Vitamin E can also be used for people who have NASH and don't have diabetes or cirrhosis.

One can also cite obethicholic acid which improves the histological features of non-alcoholic steatohepatitis. (Neuschwander-Tetri et al, Lancet. 2015 Mar 14;385(9972):956-65)

Other drugs can also be used, such as ethyl-eicosapentanoic acid or ethylicosapentate (Clinical Trials NCT01154985), simtuzumab (GS 6624) (Clinical Trials, NCT01672866 and NCT01672879), GFT 505 (Clinical Trials NCT01694849), liraglutide (Clinical Trials NCT01237119), losartan (Clinical Trials NCT01051219), cenicriviroc (Clinical Trials NTC002217475), selonsertib and aramchol.

This method is particularly interesting in that it makes it possible to manage the follow-up of the patient, and the level of steatosis in the patient, and propose further diagnosis tests (such as biopsy) only to a fraction of patients that are not improving over time.

The following examples are meant to describe an aspect of invention, but shall not be limiting the invention.

## DESCRIPTION OF THE FIGURES

Figure 1: Populations used for construction, validation and target.

Figure 2: Non-inferiority of SteatoTest-2 compared to the original SteatoTest for the diagnostic of Steatosis >= 5%.

Legends: [1]NASH patients were analyzed at 24 weeks' biopsy as steatosis <5% were absent at baseline biopsy (72 cases) according to inclusion criteria in the trial. Only 3 out of 63 cases analyzed at 24 weeks had no steatosis (<5%). Due to this very small sample size the power of the comparison is weak. For the integrated analyses all 72 cases with baseline biopsies were included.

[2]The AUROCs were highly decreased by a spectrum effect in chronic hepatitis C, as cases with minimal steatosis (1-4%) represented 732/918 (80%) of S0 as defined by CRN score. Unfortunately, these minimal steatosis grades were not described in metabolic disease.

[3]The AUROCs were increased by a spectrum effect of the control group as the blood donors had much lower steatosis tests medians than controls with biopsy, increasing the specificity. The prevalence of steatosis was not dramatically increased.

[4]The AUROCs were highly impacted by a dramatic increase of the prevalence of controls, despite their higher tests medians, as the prevalence of steatosis was divided by two in comparison with the integrated population with biopsies.

[5]Adding the blood donor's subset did not change significantly the AUROCs.

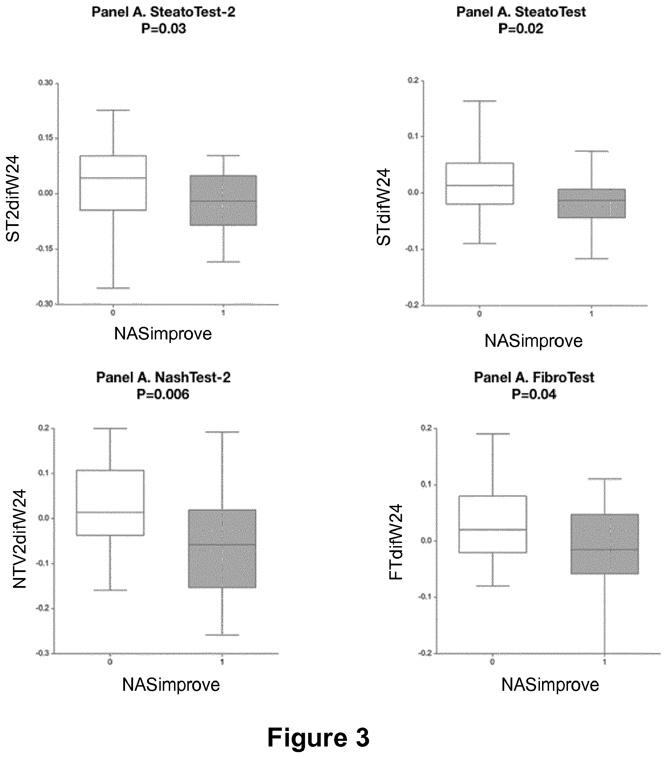

**Figure 3:** Decrease of steatosis, NASH and fibrosis biomarkers in the selonsertib trial. SteatoTest-2 decrease by -0.02 vs +0.04 (P=0.03), SteatoTest -0.014 vs +0.014 (P=0.02), NashTest-2 -0.06 vs +0.01, and FibroTest -0.02 vs +0.02 (P=0.04), in patients who improved NAS vs those who did not improved at 24 weeks.

## EXAMPLES

### Example 1. Summary

**Methods.** Five different subsets of 2,997 patients with biopsies were evaluated for test construction and validation, and four to assess the prevalence of steatosis in target populations with increasing risks of steatosis. The performance of the SteatoTest-2 was compared to the reference test, using the non-inferiority test (0.10 margin) and the Lin concordance coefficient.

**Results.** The AUROCs of the SteatoTest-2 were non-inferior to the reference test (P<0.001). AUROCs varied in the SteatoTest-2 and the reference test according to subsets and the prevalence of steatosis, with 0.772 (95%CI 0.713-0.820) vs 0.786 (0.729-0.832) in the 2,997 cases with biopsy and 0.822 (0.810-0.834) vs 0.868 (0.858-0.878) in the 5,776 cases including healthy subjects without risk factors of steatosis as controls, respectively. The Lin coefficient was highly concordant (P<0.001), from 0.74 (0.74-0.74) in presumed NAFLD to 0.91(0.89-0.93) in the construction subset.

**Conclusions.** The SteatoTest-2 is simpler and non-inferior to the first generation SteatoTest for the diagnosis of steatosis, without the limitations of body mass index and bilirubin.

### Example 2. PATIENTS AND METHODS

#### Study design

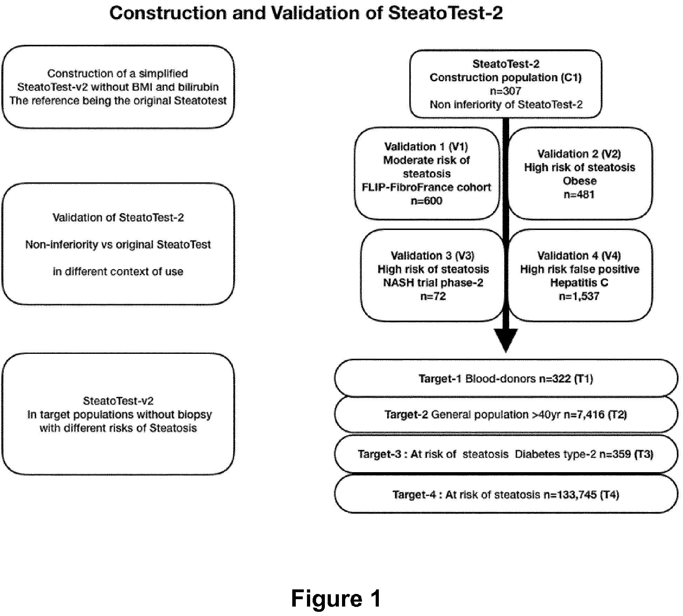

The primary aim of the study that prospectively analyzed patient subsets (**Figure 1**), was to construct a new SteatoTest-2 that was simplified, highly concordant with and not inferior to the first generation SteatoTest, with greater applicability and less risk of false positives in the general population. The SteatoTest-2 was also assessed in a prospective trial of selonsertib for the treatment of NASH based on paired biopsies before and after treatment.

The secondary aim was to assess the performance of SteatoTest-2 when combined with NashTest-2 as disclosed in WO2018050804, in non-invasive algorithms reproducing the histological NASH-algorithms (CRN or FLIP), which needed the presence of steatosis for the diagnostic of NASH (Poynard al, Eur J Gastroenterol Hepatol. 2018;30:384-391 ; Poynard et al, Eur J Gastroenterol Hepatol. 2018;30:569-577).

The variability of SteatoTest-2 AUROCs was evaluated in relation to the prevalence of steatosis, the spectrum effect, degrees of inflammation, stages of fibrosis, fasting glucose, and BMI. Finally, the SteatoTest-2 was validated for the CRN-grades of moderate and marked steatosis (European Association for the Study of the Liver (EASL); European Association for the Study of Diabetes (EASD); European Association for the Study of Obesity (EASO). EASL-EASD-EASO Clinical Practice Guidelines for the management of non-alcoholic fatty liver disease. J Hepatol. 2016;64:1388-402).

#### Patients and controls

A total of nine different subsets of individual data were included. All data were previously published (Figure 1). The first was the construction subset of the original test (C1) including 307 cases with different causes of steatosis, and controls (6. Poynard et al. Comp Hepatol 2005; 4:10). Four subsets were used for validation (V1) including 600 patients with NAFLD (Munteanu et al., Aliment Pharmacol Ther 2016; 44:877-889); (V2) 481 obese patients (Poynard et al, PLoS One. 2012;7:e30325); (V3) 72 with NASH (Loomba et al, Hepatology. 2017); and (V4) 1,537 with chronic hepatitis C (Poynard et al, J Hepatol. 2011;54:227-235). These five subsets had biopsies. Finally, four subsets without biopsies were used to assess the prevalence of steatosis presumed by both SteatoTest-2 and SteatoTest, in targeted subjects with increasing risks of steatosis; (T1) 327 blood donors (Jacqueminet et al, Clin Gastroenterol Hepatol. 2008;6:828-31); (T2) 7,416 healthy volunteers (Poynard et al, BMC Gastroenterol 2010; 10:40); (T3) 359 type2-diabetics (Jacqueminet et al, *op. cit.*) and (T4) 133,045 patients with NAFLD (Munteanu et al, 2016, *op. cit.*). Controls with a low or very low risk are described in the statistics section.

## Histological references

All biopsies were scored by experienced pathologists, blinded to historical biopsy reports, test results, and other clinical data. The FLIP-CRN scoring system, known in the art, was used for the main endpoint. The steatosis score (S) assesses and rates the quantity of large or medium-sized lipid droplets from 0 to 3, except for foamy microvesicles, (S0: <5%; S1: 5-33%, mild; S2: >33-66%, moderate; S3: ≥66%, marked). Activity grade (A, from 0 to 4) is the unweighted addition of hepatocyte ballooning (0-2) and lobular inflammation (0-2). Cases with A0 (A = 0) have no activity; A1(A = 1) mild activity; A2 (A = 2) moderate activity; A3 (A = 3) severe activity and A4 (A = 4) very severe activity. Fibrosis stage (F) was assessed by the following score: stage 0 (F0) = none; stage 1 (F1) = 1a or 1b perisinusoidal zone 3 or 1c portal fibrosis; stage 2 (F2) = perisinusoidal and periportal fibrosis without bridging; stage 3 (F3) = bridging fibrosis and stage 4 (F4) = cirrhosis. To reduce interobserver variability and standardize the reading based on the new SAF-FLIP histological classification, reports reviewed by members of the FLIP Pathology Consortium (Frederic Charlotte, for C1, V1, V2, Pierre Bedossa for C1, V1, V4 Dina Tianakos for V1) or CRN (Zack Goodman for V1, and V3) were used.

## Blood tests

The FibroTest, ActiTest and original SteatoTest are patented as 'In Vitro Diagnostic Multivariate Index Assays' for the diagnosis of METAVIR fibrosis stages, including cirrhosis, for SAF-equivalent activity and for SAF-equivalent steatosis grades, respectively (Bedossa et al, Hepatology 2012; 56: 1751-9). A quantitative NashTest-2 was constructed and internally validated in 1081 patients at risk of metabolic liver disease (Poynard et al, Eur J Gastroenterol Hepatol. 2018;30:569-577). These tests are exclusively available online and include clinical security algorithms. The recommended cutoffs were the same, whatever the chronic liver disease. Analyzers and kits were those validated and recommended by BioPredictive, and all controls assays were performed in the reference biochemistry department of Pitié Salpêtrière Hospital.

The original SteatoTest, recommended in recent NAFLD guidelines, was used as the comparator (European Association for the Study of the Liver (EASL), *op. cit.*). The SteatoTest-2 was constructed by regression analysis without BMI or total bilirubin as components, but with AST. Thus, the new test included the following ten components in its patented formula: alpha2-macroglobulin, apolipoprotein A1, haptoglobin, GGT, ALT, AST, total cholesterol, and fasting glucose, adjusted by age and gender.

## Statistical analysis

The protocol and analyses followed FibroSTARD recommendations adapted for steatosis.The limitations of the FLIP and CRN standard definitions of metabolic liver disease and their impact on the construction of non-invasive tests were previously discussed (Poynard et al, Eur J Gastroenterol Hepatol. 2018;30:384-391; Poynard et al, Eur J Gastroenterol Hepatol. 2018;30:569-577). These limitations include the presence of appropriate histological controls in only 2.2% (13/576) of cases without steatosis and without inflammatory activity in the reference study of the CRN-group.

SteatoTest-2 was constructed retrospectively (C1) based on the same subset used for the construction of the original SteatoTest, from a sample size of 307 cases with all components. To ensure appropriate power for the non-inferiority tests between the AUROCs of the new test and the reference test, as well as for the correlation of concordance in the different data subsets, more than 2000 subjects at risk of metabolic or virologic steatosis, with centralized biopsies were included. Two validation subsets, V3 and V4, had not been previously published for assessing the performance of SteatoTest.

For the main endpoint of non-inferiority, empirically estimated AUROCs were directly compared, without using non-binary-AUROCs because the same patients underwent both the new and reference tests simultaneously in each data subset. The primary endpoint was to compare test results in all included cases with biopsies for the diagnosis of all grades of steatosis (from S1 to S3 CRN grades) to the absence of steatosis (S0).

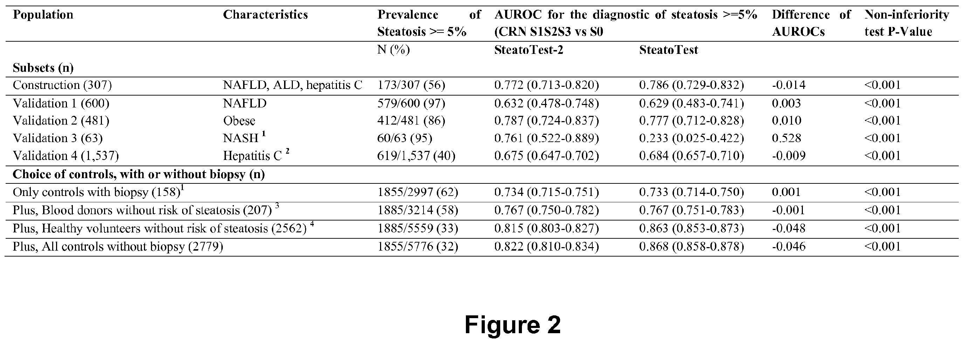

To prevent the limitations of previous studies of biomarkers, which have included fewer than 30 cases without steatosis, a large number of controls were discussed, 158 cases with histologically proven grade CRN-S0 from validation subsets (V1-V4), and 2,779 controls of T1 (=207) and T2 (n=2,562) subsets without biopsy (Figure 2). These controls without biopsy, were defined on the same criteria than those used for the controls of studies which assessed the performances of localized proton magnetic resonance spectroscopy (MRS), by hepatic triglyceride content (HTGC): no identifiable risk factors, a BMI below 25 kg/m$^2$, no diabetes, fasting glucose below 6.1 mmol/L, minimal alcohol consumption (20g for women, 30 g for men) and no known liver disease. Secondary endpoints were AUROCs with specificity assessed in three control groups, biopsy+T1-controls, biopsy+T2-controls and biopsy+T1+T2-controls)

Cutoffs were based on the method used for MRS. In that study, the 95% percentile of HTGC, assessed in 345 controls with no risk of steatosis, was 5.56%. This corresponds to a hepatic level of 55.6 mg/g and is considered the cutoff for the upper limit of normal (ULN) on MRS, and a reference for the absence of steatosis (grade S0). In our study a cutoff was chosen that optimized a high negative predictive value (at least 90%) of ST2 for the diagnosis of steatosis ≥5%. Based on the usual range of the prevalence of steatosis (17-46%) in adults, it was chosen 18.1% (95%CI 17.2%-18.9%; 1336/7395) as the predetermined prevalence to determine the negative predictive value of the new cutoff of SteatoTest-2. This prevalence was previously assessed in consecutive healthy volunteers, representative of the French population aged 40 years or older.

The performances of SteatoTest-2 were compared to the reference SteatoTest in the C1 to V4 subsets, using the non-inferiority test (0.10 margin) of the difference between AUROCs predicting NAS-CRN steatosis grades 1-to-3 versus grade 0 (no steatosis or less than 5% of hepatocytes). Sensitivity analyses were performed to evaluate the influence of factors of variability (inflammation grade, fibrosis stage, obesity, and fasting glucose with two cutoffs 6.1 for insulino resistance and 7.0 for diabetes type-2).

To ensure that the prediction of the grades of steatosis was similar in all the subsets by the Steatotest-2 and the SteatoTest, the level and significance of the Lin concordance coefficient was assessed between the two tests.

The medians and the interquartile distribution of all tests were graphically represented according to histological scores and control subsets. The Tukey-Kramer's test compared all pairs simultaneously with confidence intervals of mean differences and P-Values. Notched box plots were constructed using the formula: median $\pm(1.57\text{x}IQR/\sqrt{n})$. If the notches of two boxes did not overlap, the medians were significantly different.

In patients in the selonsertib trial, liver biopsies, and serum markers including the SteatoTest, SteatoTest-2, NashTest-2, and FibroTest, were performed at baseline and at 24 weeks of treatment. The differences in paired test results at baseline and at 24 weeks of treatment were compared between patients without (nonresponders) and with (responders) a histological improvement, defined as ≥1-point improvement in the NAFLD Activity Score. All analyses were blinded to treatment effect as set out in the protocol.

The influence of the definition of steatosis (presumed by the SteatoTest2) was assessed on the prevalence of NASH, presumed by the NashTest-2 in the subsets of patients without biopsies, based on the standard CRN algorithm (steatosis ≥5%) or the FLIP algorithm for significant activity (FLIP score A2) as previously published. The aim, for use in large populations, was to identify a sensitive cutoff for the SteatoTest-2 for subjects with at least steatosis grade S1 and to identify those cases with clinically significant NASH, that is, at least grade N2 in the CRN or the FLIP scoring system.

All statistical analyses were performed using NCSS-12.0 and R.

*Example 3. RESULTS*

**Characteristics of included subjects**

A total of 2,997 patients with biopsies and histological scores were assessed by the SAF scoring system. Although the characteristics of included subjects have already been published in previous publications, the value of the present integrated database was the wide range of characteristics, making it possible to evaluate the robustness of the blood tests according to factors of variability. The IQR of age was between 34 and 61 years old. The prevalence of histological steatosis ranged from 7.3% to 24.5%, NASH from 13.7% to 100%, cirrhosis from 0% to 42.6%, the prevalence of T2-diabetes from 7.5% to 70.8%, and obesity (BMI≥30) from 13.6% to 100%. The subset of obese subjects was younger, with a higher percentage of women, and a lower prevalence of advanced fibrosis than the other subsets.

**New SteatoTest-2**

Unlike the SteatoTest, the SteatoTest-2 did not include BMI or total bilirubin, but did include AST, with different independent coefficients for the remaining nine components.

The clinical characteristics and grades of steatosis and NASH as well as the stages of fibrosis presumed by the blood tests in the four targeted subsets with increasing risks of steatosis were recorded. As expected, the prevalence of steatosis presumed by the SteatoTest increased from 15.5% in blood donors to 84.4% in subjects who underwent a FibroTest.

The AUROCs of the SteatoTest-2 were non-inferior to the reference SteatoTest, which was the comparator. All non-inferiority tests were significant (P<0.001)(Figure 2). Results of the AUROCs for the SteatoTest -2 and SteatoTest varied depending on the control groups and the prevalence of steatosis: 0.772 (95%CI 0.713-0.820) and 0.786 (0.729-0.832), respectively in the 2,997 cases with biopsy (prevalence of steatosis 62%) to 0.822 (0.810-0.834) and 0.868 (0.858-0.878), respectively in the 5,776 cases including controls without risk factors of steatosis and without biopsy (prevalence of steatosis 32%),(Figure 2).

The influence of the selection of controls on SteatoTest-2 AUROCs was evaluated. AUROCs varied from 0.734 (95%CI 0.715-0.751) with controls-S0-biopsy to 0.822 (0.810-0.834) with controls-S0-biopsy-T1-T2. As the spectrum of cases with steatosis were always defined by biopsy (stages S1 to S3), the sensitivity of the test did not vary. Therefore, the AUROCs were influenced by both the spectrum of the control group which was directly related to the test specificity, but also by the change in the prevalence of steatosis which varied from 32 to 62%. When controls-T1 (n=207, median SteatoTest-2=0.15) were added to the controls-SO-biopsy, the AUROC increased slightly from 0.734(0.715-0.751) to 0.767(0.750-0.782), and the prevalence of steatosis decreased from 62% to 58%. When controls-T2 (n=2,562 median SteatoTest-2=0.30) were added to the controls-S0-biopsy, there was both a marked increase in the AUROC 0.815 (0.803-0.827) as well as a marked decrease in steatosis prevalence from 62% to 34%.

The Lin coefficient was highly concordant (P<0.001), in all subsets and was between 0.74 (0.74-0.74) in A4 and 0.91 (0.89-0.93) in C1.

**Cutoffs for the SteatoTest-2**

The upper 95% percentile (ULN) in the Steato-Test-2 was 0.40 in 177 blood donors with BMI<25 and fasting glucose <6.1. This cutoff was chosen as the ULN for ST2 to predict the presence of at least 5% of steatosis. The following cut offs were recommended according to median values and 95% confidence intervals : S1>=0.40, S2>=0.55, S3 >=0.62.

The sensitivity for the integrated database with biopsies using the 0.40 cutoff was 79% (77-85) with a 92% predictive value, when adjusted for the predetermined prevalence of 18%. The corresponding specificity was 50% (47-53).

**Comparison between presumed prevalences of NASH according to CRN or FLIP simplified algorithms**

The prevalence of steatosis in the NAFLD subset, was 84.7%. The prevalence of clinically significant NASH (moderate or severe) was 72.3% (434/600) using the CRN-algorithm and 80.3% (482/600) using the FLIPA2-algorithm, which was significantly (P=0.001) 8.0% (95%CI 3-13%) different.

The prevalence of steatosis in the subset of obese subjects, was 65.5%. The prevalence of clinically significant NASH was 16.6% (80/481) using the CRN-algorithm and 18.1% (87/481) using the FLIP-A2-algorithm, which was non-significantly 1.5% (-4%;6%; P=0.55) different.

All included patients in the NASH trial subset had histological steatosis and NASH at inclusion, whatever the CRN or FLIP-simplified definition, and all had presumed steatosis by the SteatoTest-2.

The prevalence of steatosis in the blood donor subset was 15.5%. The prevalence of clinically significant NASH (moderate or severe) was 3.1% (10/322) using the CRN-algorithm and 5.3% (17/322) using the FLIPA2-algorithm, which was a non-significant (P=0.17) 2.2% (-1.2;6.8%) difference.

The prevalence of steatosis in the subset of healthy volunteers was 50.1%. The prevalence of clinically significant NASH (moderate or severe) was 22.5% (1,667/7,416) using the CRN-algorithm and 29.9% (2,220/7,416) using the FLIPA2-algorithm, which was a significant (P<0.001) 7.5% (6.0;8.9%) difference.

The prevalence of steatosis in the subset of patients with diabetes was 85.2%. The prevalence of clinically significant NASH (moderate or severe) was 41.8% (150/359) using the CRN-algorithm and 44.6% (160/359) using the FLIPA2-algorithm, which was a non-significant (P=0.45) 2.8% (-4.7;10.3%) difference.

**SteatoTest-2, NashTest-2, FibroTest improvements in the selonsertib trial (Figure 3)**

All 72 patients with NASH and stage 2 (35%) or 3 (65%) fibrosis were included. At baseline, all patients had a NAS score $\geq$5 (100%). At 24 weeks, NAS improved by at least one point in 49% of patients. The SteatoTest-2 decreased by - 0.02 vs +0.04 (P=0.03)(Figure 3.A) in patients who improved compared to those who did not, respectively, the SteatoTest by -0.014 vs +0.014 (P=0.02)(Figure 3.B), the NashTest-2 -0.06 vs +0.01 (Figure 3.C), and FibroTest -0.02 vs +0.02 (P=0.04) (Figure 3.D). Despite the narrow range of NASH disease severity (only grade 2 and 3), the AUROC for predicting an improvement in NAS by NashTest-2 was 0.700 (95% CI 0.543, 0.809; p=0.003).

**Sensitivity analyses**

The AUROCs of SteatoTest-2 were non-inferior to the reference SteatoTest, whatever the associated inflammatory activity, fibrosis stage, fasting glucose level,or obesity. The only exception was the small subset of patients with hepatitis C without activity, in which only 11 cases had steatosis. Statistical comparisons were not possible in two subsets due to a 98% prevalence of steatosis, that is, in obese patients with advanced fibrosis and with fasting glucose >= 7 mmol/L.

**Association with grades of CRN steatosis.**

Significant differences were found in all different stages of disease by histological grade with the SteatoTest-2. The medians were 0.40, 0.53, 0.60 for S0, S1, S2, and S3 respectively.

*Example 4. Discussion*

In this study is described the construction and characterization of a new blood test for the diagnosis of steatosis and the validation that its results are not inferior to the reference SteatoTest, a recognized comparator (European Association for the Study of the Liver, *op. cit.*). This new test has the advantage of not including body mass index or total bilirubin in its components, which are two causes of significant variability.

**Non-inferiority and concordance with the comparator**

It is confirmed that Steato-test2 was non-inferior to the comparator for the most frequent causes of liver steatosis, in patients with metabolic liver disease (overweight, diabetes type 2, dyslipidemia) as well as in those with chronic hepatitis C, with biopsies. To evaluate its specificity, it was also demonstrated the non-inferiority of this new test in subsets with a lower risk of steatosis, including the general population and very low risk groups such as blood donors, healthy volunteers and hepatitis C without steatosis. Furthermore, the highly significant quantitative concordance between these two tests was confirmed in all subsets. The comparison of AUROCs demonstrated non-inferiority after the major sources of variability were taken into account, in particular, the prevalences of steatosis, stages of fibrosis, grades of inflammatory activity as well as the prevalence of diabetes, obesity, and increase in fasting glucose with standard cutoffs (6.1 and 7.0 mmol/L).

**Choices and impact of controls**

This study emphasized the importance of control (without steatosis) and case (with steatosis) selection in studies evaluating the AUROCs of biomarkers. The construction of this new test took into account the methodological limitations of previous studies, concerning sample size as well as the definitions of steatosis and controls and their impact on AUROCs.

Due to the ethical limitations of performing biopsies in healthy controls or in cases with metabolic liver disease and normal liver function tests, the same inclusion criteria as those used for MRS were used as our reference to define the presence or absence of steatosis and define the ULN in SteatoTest-2. It is felt that these criteria should be recommended in guidelines to standardize the methodology when constructing steatosis biomarkers, thus preventing artificial disagreement.

The selection of patients with steatosis in whom biopsy is indicated based on MRS or not, can significantly influence the AUROCs in biomarker studies. If a study is designed with MRS first, then biopsy is only performed in subjects with steatosis on MRS, and controls to evaluate specificity will be those patients with steatosis on MRS but not on biopsy. This is obviously rare and does not correspond to a frequent context of use. Thus, these studies cannot reliably assess specificity and AUROCs for steatosis. This strategy is also questionable to assess the value of NASH biomarkers because ballooning and lobular inflammation may be present with or without steatosis <5% at biopsy. Due to these limitations, it was decided to assess the specificity of steatosis biomarkers in large samples. Like the first generation SteatoTest, all available biopsies from 498 patients with chronic viral hepatitis were included, as well as 1,537 subjects with no risk of steatosis as controls, using the ULN for MRS as the basis of our ULN (Szczepaniak et al, Am J Physiol Endocrinol Metab. 2005;288:462-8).

In this study the influence of the arbitrary selection of controls when evaluating steatosis non-invasive tests by AUROCs was demonstrated. Without changing the test sensitivity AUROCs "artificially" varied from 0.734 with biopsy-controls to 0.822 with biopsy+T1+T2-controls. The results were influenced by both the spectrum of the control group, but also by the change in the prevalence of steatosis varying from 32 to 62% according to the selection of cases without steatosis. As the prevalence of steatosis varies between 20 and 50%, presumed by MRS, the performances of new biomarkers of steatosis must be assessed according to these sources of variability, and avoiding indirect comparison of AUROCs.

**Performance for marked (grade S2) and severe (S3) CRN grades of steatosis**

It was also shown, for the first time, that SteatoTest-2 significantly discriminates among the four stages of the CRN score. Although the semi-quantitative correlations and significant differences among histological stages in our study are not similar to the results of MRS, which has a better correlation with percentages of hepatocyte steatosis than any blood test, the aim was to validate a robust test without the practical limitations of MRS. While the main value of SteatoTest-2 is its sensitivity and associated high negative predictive value (92%) for the diagnosis of steatosis of at least 5%, there was also a significant correlation between test values and histological grades of steatosis. Furthermore, the clinical value of the AUROC (0.603;0.577-0.229; P<0.001 vs random) for the diagnosis of S2S3 vs S1 is limited. Indeed, this AUROC was assessed in a narrow range of steatosis spectrum, without S0 controls, or controls with no risk of steatosis.

**Validation in NASH paired biopsies (Figure 3)**

It was demonstrated for the first time in a phase-2 trial of selonsertib, that the changes in the three non-invasive tests, SteatoTest-2, NashTest-2 and FibroTest, can reliably predict improvement in the NAS score in patients with NASH. This confirmed that the paired SteatoTest-2 detected an improvement in steatosis in patients with moderate and severe grades of steatosis, which was also observed in biopsies and MRS.

Despite the retrospective design, the SteatoTest-2 was clearly non-inferior to and highly concordant with the reference SteatoTest which has been extensively used and validated by guidelines for the diagnostic of steatosis.

In conclusion, the new multi-analyte SteatoTest-2 simplifies, and was found to be non-inferior to, the reference SteatoTest for the diagnosis of steatosis, without the variability induced by body mass index and the risk of false positives related to unconjugated bilirubin.

**Claims**

1. An *in vitro* method for diagnosing the presence of liver steatosis in a subject comprising the steps of

    (a) combining the values of at least three biological markers, selected from the group consisting of:

        ◦ the amount of biochemical markers as measured circulating in the blood, serum or plasma, of the subject, and
        ◦ physical characteristics of a patient
        in a function, in order to obtain an end value,

    (b) optionally comparing the end value to predetermined values, and
    (c) determining presence of STEATOSIS based on the end value calculated on (a),
    with the proviso that Body Mass Index and level of bilirubin in the subject are not used as biological markers in (a).

2. The *in vitro* method of claim 1, wherein the circulating biochemical markers are selected from the group consisting of $\alpha$2-macroglobulin (A2M), GGT (gammaglutamyl transpeptidase), haptoglobin, apolipoprotein A-I (apoA1), alanine transaminases (ALT), aspartate transaminases (AST), triglycerides, total cholesterol, fasting glucose, $\gamma$-globulin, albumin, $\alpha$1-globulin, $\alpha$2-globulin, $\beta$-globulin, IL10, TGF-$\beta$1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1 and carbohydrate deficient transferrin.

3. The *in vitro* method of any one claim 1 to 2, wherein the function includes at least one variable chosen in the group consisting of gender and age of the subject.

4. The *in vitro* method of any one of claims 1 to 3, wherein the function includes the values measured for circulating Alpha-2-macroglobulin, ApoA1, GGT, Haptoglobin, ALT, AST, fasting glucose, total cholesterol, triglycerides, as well as the values corresponding to the age and sex of the patient.

5. The *in vitro* method of any one of claims 1 to 4, wherein the function is a logistic function obtained by logistic regression.

6. The *in vitro* method of claim 5, wherein the function has been obtained by :

   a) evaluating the presence of steatosis in a cohort of patients, wherein the values of circulating biochemical markers are known for the patients
   b) identifying by unidimensional analysis, the circulating biochemical markers for which the values differ significantly between the groups of

   i. patients with steatosis and
   ii. patients without steatosis

   c) performing a logistic regression analysis to assess and ponder the independent discriminative value of the markers identified in step b) for the occurrence of steatosis
   d) thereby obtaining the function, by combination of these identified independent factors.

7. The *in vitro* method of any one of claims 1 to 6, wherein the function is $a0 + a1 \times$ Age (years) $+ a2 \times$ ApoA1 (g/l) $+ a3 \times$ Log (A2M, g/l) $+ a4 \times$ Log(GGT, IU /l) $+ a5 \times$ Log(ALT, IU /l) $+ a6 \times$ Log(AST, IU /l) $+ a7 \times$ Log (Hapto, g/l) $+ a8 \times$ Log (Triglycerides TG, mmol/l) $+ a9 \times$ Log (Total Cholesterol CT, mmol/l) $+ a10 \times$ Log (Fasting glucose, g/l) $+ a11 \times$ Gender (0 for women, 1 for men).

8. The *in vitro* method of claim 7, wherein

   a) $4.8 \leq a0 \leq 5.5$ preferably $5 \leq a0 \leq 5.3$
   b) $-0.03 \leq a1 \leq -0.015$ preferably $-0.025 \leq a1 \leq -0.02$
   c) $0.9 \leq a2 \leq 1.2$ preferably $0.95 \leq a2 \leq 1.05$
   d) $1.8 \leq a3 \leq 2.2$ preferably $1.95 \leq a3 \leq 2.05$
   e) $-1.3 \leq a4 \leq -1.1$ preferably $-1.2 \leq a4 \leq -1.1$
   f) $-1.45 \leq a5 \leq -1.2$ preferably $-1.36 \leq a5 \leq -1.3$
   g) $0.5 \leq a6 \leq 0.7$ preferably $0.55 \leq a6 \leq 0.65$
   h) $-0.35 \leq a7 \leq -0.22$ preferably $-0.3 \leq a7 \leq -0.25$
   i) $-1.45 \leq a8 \leq -1.25$ preferably $-1.4 \leq a8 \leq -1.3$
   j) $-0.8 \leq a9 \leq -0.6$ preferably $-0.75 \leq a9 \leq -0.65$
   k) $-3.55 \leq a10 \leq -3.35$ preferably $-3.5 \leq a10 \leq -3.4$
   l) $0.35 \leq a11 \leq 0.55$ preferably $0.4 \leq a11 \leq 0.5$.

9. The *in vitro* method of any one of claims 1 to 8, wherein the function is $5.17 - 0.022 \times$ Age (years) $+ 1.02 \times$ ApoA1 (g/l) $+ 1.99 \times$ Log (A2M, g/l) $- 1.16 \times$ Log(GGT, IU / l) $- 1.33 \times$ Log(ALT, IU / l) $+ 0.6 \times$ Log(AST, IU / l) $- 0.29 \times$ Log (Hapto, g/l) $- 1.35 \times$ Log (Triglycerides TG, mmol/l) $- 0.68 \times$ Log (Total Cholesterol CT, mmol/l) $- 3.46 \times$ Log (Fasting glucose, g/l) $+ 0.46 \times$ Gender (0 for women, 1 for men).

10. The *in vitro* method of claim 9, wherein the predetermined value is 0.25, and wherein the patient has nonalcoholic steatohepatitis (steatosis) if the end result is higher or equal to 0.25, and does not have nonalcoholic steatohepatitis (steatosis) if the end result is below 0.25.

11. A method for obtaining a function for identifying the presence of steatosis in a patient wherein said function combines the values of the concentration of biochemical markers in the blood / serum or plasma of said patient and optionally the age, gender of the patient, comprising the steps of:

   a) evaluating the presence of steatosis in a cohort of patients, wherein the values of circulating biochemical markers are known for the patients
   b) identifying by unidimensional analysis, the circulating biochemical markers for which the values differ significantly between the groups of

   i. patients with steatosis and
   ii. patients without steatosis

c) identifying whether the age, gender of the patient differ significantly between the groups of

    i. patients with steatosis and
    ii. patients without steatosis

d) performing a logistic regression analysis to assess and ponder the independent discriminative value of the markers identified in step b) and c) for the occurrence of steatosis, wherein Body Mass Index and level of bilirubin are not incorporated in the list of markers from which the logistic regression is performed.

e) thereby obtaining the function, by combination of these identified independent factors, wherein the function allows for diagnosing the presence of nonalcoholic steatohepatitis (steatosis) in a subject and doesn't use the values of bilirubin or BMI.

12. The method of claim 11, wherein the biochemical markers of step b) are selected from the group consisting of α2-macroglobulin (A2M), GGT (gammaglutamyl transpeptidase), haptoglobin, apolipoprotein A-I (apoA1), alanine transaminases (ALT), aspartate transaminases (AST), triglycerides, total cholesterol, fasting glucose, γ-globulin, albumin, α1-globulin, α2-globulin, β-globulin, IL10, TGF-β1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1, carbohydrate deficient transferrin, α-fetoprotein (AFP), fucosylated AFP, HSP27 (heat shock protein), HSP70, Glypican-3 (GPC3), squamous cell carcinoma antigen (SCCA) and in particular SCCA-IgM IC which is a circulating immune complex composed of SCCA and IgM, Golgi protein 73 (GP73), α-L-fucosidase (AFU), Des-γ-carboxyprothrombin (DCP or PIVKA), Osteopontin (OPN), and Human Carbonyl Reductase.

13. An *ex vivo* method for diagnosis a liver disease in a patient, comprising the steps of

    - Performing the method of any one of claims 1 to 10 to determine presence of steatosis in a patient
    - Determining the presence of liver fibrosis in the patient.
    - Determining the presence of liver inflammation in the patient, and diagnosing a liver disease in the patient depending on the results of the above determinations.

14. The method of claim 13, wherein liver fibrosis is determined by the combination of the values biological markers in a function, in particular by the Fibrotest, or by *ex vivo* measurement of liver stiffness by Vibration Controlled Transient Elastography.

15. The method of claim 13 or 14, wherein presence of liver inflammation in the patient is determined by the combination of the values biological markers in a function, in particular by the NASH test.

## Construction and Validation of SteatoTest-2

Figure 1

| Population | Characteristics | Prevalence of Steatosis >= 5% | AUROC for the diagnostic of steatosis >=5% (CRN S1S2S3 vs S0 | | Difference of AUROCs | Non-inferiority test P-Value |
|---|---|---|---|---|---|---|
| | | N (%) | SteatoTest-2 | SteatoTest | | |
| **Subsets (n)** | | | | | | |
| Construction (307) | NAFLD, ALD, hepatitis C | 173/307 (56) | 0.772 (0.713-0.820) | 0.786 (0.729-0.832) | -0.014 | <0.001 |
| Validation 1 (600) | NAFLD | 579/600 (97) | 0.632 (0.478-0.748) | 0.629 (0.483-0.741) | 0.003 | <0.001 |
| Validation 2 (481) | Obese | 412/481 (86) | 0.787 (0.724-0.837) | 0.777 (0.712-0.828) | 0.010 | <0.001 |
| Validation 3 (63) | NASH [1] | 60/63 (95) | 0.761 (0.522-0.889) | 0.233 (0.025-0.422) | 0.528 | <0.001 |
| Validation 4 (1,537) | Hepatitis C [2] | 619/1,537 (40) | 0.675 (0.647-0.702) | 0.684 (0.657-0.710) | -0.009 | <0.001 |
| **Choice of controls, with or without biopsy (n)** | | | | | | |
| Only controls with biopsy (158)[1] | | 1855/2997 (62) | 0.734 (0.715-0.751) | 0.733 (0.714-0.750) | 0.001 | <0.001 |
| Plus, Blood donors without risk of steatosis (207) [3] | | 1885/3214 (58) | 0.767 (0.750-0.782) | 0.767 (0.751-0.783) | -0.001 | <0.001 |
| Plus, Healthy volunteers without risk of steatosis (2562) [4] | | 1885/5559 (33) | 0.815 (0.803-0.827) | 0.863 (0.853-0.873) | -0.048 | <0.001 |
| Plus, All controls without biopsy (2779) | | 1855/5776 (32) | 0.822 (0.810-0.834) | 0.868 (0.858-0.878) | -0.046 | <0.001 |

# Figure 2

Figure 3

Europäisches
Patentamt

European
Patent Office

Office européen
des brevets

# EUROPEAN SEARCH REPORT

**Application Number**

EP 18 30 6024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/082603 A1 (CALES P. [FR] ET AL.) 23 March 2017 (2017-03-23) * abstract * * claims 1,4-7,10-11,13-14 * | 1-3,5,6, 11-14 | INV. G16H50/20 G16H50/30 G01N33/68 |
| X | WO 2014/049131 A1 (UNIVERSITÉ D'ANGERS [FR]) 3 April 2014 (2014-04-03) * abstract * * page 8, line 7 - line 15 * * page 9, line 1 - line 28 * * page 11, line 1 - page 14, line 5 * * claims 1-15 * | 1-3,5,6, 11-15 | |
| Y,D | WO 2018/050804 A1 (BIOPREDICTIVE [FR]) 22 March 2018 (2018-03-22) * abstract * * claims 11-15 * | 4 | |
| Y,D | POYNARD T. ET AL.: "Applicability and precautions of use of liver injury biomarker FibroTest", BMC GASTROENTEROL., vol. 11, no. 1, 39, 14 April 2011 (2011-04-14), pages 1-11, XP021098125, * abstract * * page 4, column 1, paragraph 3 * | 4 | |
| A | MUNTEANU M. ET AL.: "Diagnostic performance of FibroTest, SteatoTest and ActiTest in patients with NAFLD using the SAF score as histological reference", ALIMENT. PHARMACOL. THER., vol. 44, no. 8, 23 August 2016 (2016-08-23), pages 877-889, XP055327194, * the whole document * | 1-15 | TECHNICAL FIELDS SEARCHED (IPC) G16H G01N |

The present search report has been drawn up for all claims

| Place of search | Date of completion of the search | Examiner |
|---|---|---|
| Munich | 26 October 2018 | Giry, Murielle |

## ANNEX TO THE EUROPEAN SEARCH REPORT
## ON EUROPEAN PATENT APPLICATION NO.

EP 18 30 6024

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2018

| Patent document cited in search report | Publication date | Patent family member(s) | Publication date |
|---|---|---|---|
| US 2017082603 A1 | 23-03-2017 | NONE | |
| WO 2014049131 A1 | 03-04-2014 | NONE | |
| WO 2018050804 A1 | 22-03-2018 | EP 3296744 A1<br>WO 2018050804 A1 | 21-03-2018<br>22-03-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0216949 A

- WO 2018050804 A

**Non-patent literature cited in the description**

- *J Hepatol,* 2015
- **POYNARD et al.** *Comparative Hepatology,* 2005, vol. 4, 10
- **ANGULO et al.** *Hepatology,* 2007, vol. 45 (4), 846-854
- **FEDCHUK et al.** *Aliment Pharmacol Ther,* 2014, vol. 40, 1209-1222
- **BUTLER et al.** *Clinical Nutrition ESPEN,* 2017, vol. 22, 112e115
- **SORKIN et al.** *American Journal of Epidemiology,* 1999, vol. 150 (9), 969-977
- **POYNARD et al.** *BMC Gastroenterol.,* 2011, vol. 11, 39
- **NEUSCHWANDER-TETRI et al.** *Lancet,* 14 March 2015, vol. 385 (9972), 956-65
- **POYNARD.** *Eur J Gastroenterol Hepatol.,* 2018, vol. 30, 384-391
- **POYNARD et al.** *Eur J Gastroenterol Hepatol.,* 2018, vol. 30, 569-577
- *J Hepatol.,* 2016, vol. 64, 1388-402
- **POYNARD et al.** *Comp Hepatol,* 2005, vol. 4, 10
- **MUNTEANU et al.** *Aliment Pharmacol Ther,* 2016, vol. 44, 877-889
- **POYNARD et al.** *PLoS One.,* 2012, vol. 7, e30325
- **LOOMBA et al.** *Hepatology,* 2017
- **POYNARD et al.** *J Hepatol.,* 2011, vol. 54, 227-235
- **JACQUEMINET et al.** *Clin Gastroenterol Hepatol.,* 2008, vol. 6, 828-31
- **POYNARD et al.** *BMC Gastroenterol,* 2010, vol. 10, 40
- **BEDOSSA et al.** *Hepatology,* 2012, vol. 56, 1751-9
- **POYNARD et al.** *Eur J Gastroenterol Hepatol.,* 2018, vol. 30, 384-391
- **SZCZEPANIAK et al.** *Am J Physiol Endocrinol Metab.,* 2005, vol. 288, 462-8